(19) Europäisches Patentamt
European Patent Office
Office européen des brevets

(11) Publication number: **0 315 254 A1**

# (12) EUROPEAN PATENT APPLICATION

(21) Application number: 88202395.5

(22) Date of filing: 28.10.88

(51) Int. Cl.4: C12N 15/00 , C12P 21/02 , C12P 21/00 , G01N 33/577

(30) Priority: 30.10.87 NL 8702590

(43) Date of publication of application: 10.05.89 Bulletin 89/19

(84) Designated Contracting States: AT BE CH DE ES FR GB GR IT LI LU NL SE

(71) Applicant: Stichting Katholieke Universiteit
Toernooiveld 1
NL-6525 ED Nijmegen(NL)

(72) Inventor: Martens, Gerardus Julianus Maria
Sleedoornstraat 11
NL-6523 GE Nijmegen(NL)

(74) Representative: Smulders, Theodorus A.H.J., Ir. et al
Vereenigde Octrooibureaux Nieuwe Parklaan 107
NL-2587 BP 's-Gravenhage(NL)

## (54) Protein 7B2, recombinant DNA, cDNA and mRNA, antibodies for 7B2, and a method of detecting 7B2.

(57) The invention provides recombinant DNA comprising genetic information for 7B2 protein, and a process for preparing protein 7B2 by expression of such recombinant DNA. The invention also provides antibodies with a specificity for protein 7B2 and a method of detecting 7B2 protein or mRNA, wherein such antibodies or labelled RNA or DNA probes of 7B2 are used.

FIG.1

```
                                                    5'----CGCTCCTCGGGCTGCCCCTCGGTTGACA   28

-26                 -20                                          -10                          -1
Met Val Ser Arg Met Val Ser Thr Met Leu Ser Gly Leu Leu Phe Trp Leu Ala Ser Gly Trp Thr Pro Ala Phe Ala
ATG GTC TCC AGG ATG GTC TCT ACC ATG CTA TCT GGC CTA CTG TTT TGG CTG GCA TCT GGA TGG ACT CCA GCA TTT GCT   106

 1                           10                                     20
Tyr Ser Pro Arg Thr Pro Asp Arg Val Ser Glu Ala Asp Ile Gln Arg Leu Leu His Gly Val Met Glu Gln Leu Gly
TAC AGC CCC CGG ACC CCT GAC CGG GTC TCA GAA GCA GAT ATC CAG AGG CTG CTT CAT GGT GTT ATG GAG CAA TTG GGC   184

            30                                 40                                     50
Ile Ala Arg Pro Arg Val Glu Tyr Pro Ala His Gln Ala Met Asn Leu Val Gly Pro Gln Ser Ile Glu Gly Gly Ala
ATT GCC AGG CCC CGA GTG GAA TAT CCA GCT CAC CAG GCC ATG AAT CTT GTG GGC CCC CAG AGC ATT GAA GGT GGA GCT   262

                    60                                     70
His Glu Gly Leu Gln His Leu Gly Pro Phe Gly Asn Ile Pro Asn Ile Val Ala Glu Leu Thr Gly Asp Asn Ile Pro
CAT GAA GGA CTT CAG CAT TTG GGT CCT TTT GGC AAC ATC CCC AAC ATC GTG GCA GAG TTG ACT GGA GAC AAC ATT CCT   340

    80                           90                                     100
Lys Asp Phe Ser Glu Asp Gln Gly Tyr Pro Asp Pro Pro Asn Pro Cys Pro Val Gly Lys Thr Asp Asp Gly Cys Leu
AAG GAC TTT AGT GAG GAT CAG GGG TAC CCA GAC CCT CCA AAT CCC TGT CCT GTT GGA AAA ACA GAT GAT GGA TGT CTA   418

                    110                                    120                                    130
Glu Asn Thr Pro Asp Thr Ala Glu Phe Ser Arg Glu Phe Gln Leu His Gln His Leu Phe Asp Pro Glu His Asp Tyr
GAA AAC ACC CCT GAC ACT GCA GAG TTC AGT CGA GAG TTC CAG TTG CAC CAG CAT CTC TTT GAT CCG GAA CAT GAC TAT   496

                                140                                    150                  •
Pro Gly Leu Gly Lys Trp Asn Lys Lys Leu Leu Tyr Glu Lys Met Lys Gly Gly Glu Arg Arg Lys Arg Arg Ser Val
CCA GGC TTG GGC AAG TGG AAC AAG AAA CTC CTT TAC GAG AAG ATG AAG GGA GGA GAG AGA CGA AAG CGG AGG AGT GTC   574

            160                                170                                    180
Asn Pro Tyr Leu Gln Gly Gln Arg Leu Asp Asn Val Val Ala Lys Lys Ser Val Pro His Phe Ser Asp Glu Asp Lys
AAT CCA TAT CTA CAA GGA CAG AGA CTG GAT AAT GTT GTT GCA AAG AAG TCT GTC CCC CAT TTT TCA GAT GAG GAT AAG   652

Asp Pro Glu •••
GAT CCA GAG TAA AGAGAAGATGCTAGACGAAAACCCACATTACCTGTTAGGCCTCAGCATGGCTTATGTGCACGTGTAAATGGAGTCCCTGTGAATGAC   751

AGCATGTTTCTTACATAGATAATTATGGATACAAAGCAGCTGTATGTAGATAGTGTATTGTCTTCACACCGATGATTCTGCTTTTTGCTAAATTAGAATAAGA   854

GCTTTTTTGTTTCTTGGGTTTTTAAAATGTGAATCTGCAATGATCATAAAAATTAAAATGTGAATGTCAACAATAAAAAGCAAGACTATGAAAGGCTCAGATT   957

TCTTGCAGTTTAAAATGGTGTCTGAGGTTGTACTATTTTGGCCAAGTCTGTAGAAAGCTGTCATTTGATTTTGATTATGTAGTTCATCCAGCCCTTGGGCATT   1060

GTTATACACCAGTAAAGAAGGCTGTACTCAAGAGGAGGAGCTGACACATTTCACTTGGCTGCGTCTTAATAAACATGAATGCAAGCATTGGC(A)n----3'   1152
```

EP 0 315 254 A1

Xerox Copy Centre

**Protein 7B2, recombinant DNA, cDNA and mRNA, antibodies for 7B2, and a method of detecting 7B2.**

This invention is in the field of genetic engineering and more specifically relates to the protein 7B2, which has been described in the literature.

The protein 7B2, which has a molecular mass of about 21 kDa and a pI value of 4.9, has been described by Hsi et al., FEBS Lett. 147 (1982), 261-266 and Seidah et al., Arch.Biochem.Biophys. 2225 (1983), 525-534. The protein has been isolated by them from porcine anterior pituitary and human pituitary. They have also sequenced the first 81 and 77 amino acids, respectively, from the $NH_2$ terminal, and induced rabbit antibodies against a synthetic oligopeptide consisting of the amino acids 23-39. By means of these antibodies they have detected the presence of the protein in the anterior and posterior lobe of the pituitary, and in neurons located within the supraoptic nucleus of the hypothalamus.

Similar studies have shown that the protein occurs not only in the pituitary and the central nervous system, but also in peripheral tissues, such as the gastro-intestinal and the genito-urinary tracts, endocrine glands, and, as described by Suzuki et al., J.Clin.Endocrinol.Metab. 63 (1986), 758-765, in various endocrine tumors and in plasma of patients having such tumors, in particular pancreatic tumors (insulinomas, glucagonomas, vipomas); in patients harboring small cell lung tumors, however, 7B2 protein was not detected, either in the cells or in the blood plasma. Iguchi et al., Peptides 8 (1987), 593-598,have used the same method to demonstrate immunoreactive 7B2 in cerebrospinal fluid (brain fluid) and plasma of healthy individuals and patients having Alzheimer's disease; in that study no significant differences were observed.

The function of the protein has hitherto been unknown. Speculations as regards its function vary from a growth factor (Seidah et al.) to a neurotransmitter or neuromodulator of the central nervous system (Iguchi et al.).

Further research has now not only clarified the biological function of the protein, but in addition provided a 7B2 cDNA clone, by means of which the nucleotide sequence of the messenger RNA (mRNA) coding for 7B2, and from this the total amino acid sequence of the protein has been determined. As a result it has, on the one hand, become possible to use hybridization tests for detecting 7B2 mRNA in human or animal tissues using labelled RNA or DNA probes, and on the other hand, to produce the 7B2 protein by in-vitro or in-vivo translation, to produce other monoclonal or polyclonal antibodies having a specificity for 7B2, and, in order to detect 7B2 protein in cells and body fluids, such as blood plasma, sputum, cerebrospinal fluid, urine, and the like, to carry out immunoanalytic detection methods, such as immunofluorescence, ELISA, Western blotting, immunoprecipitation/polyacrylamide gel electrophoresis, and the like with antibodies having a higher specificity or affinity than the known antibodies directed against the amino acids 23-39.

Using these new possibilities of detecting the expression of 7B2 protein, it has further surprisingly been found that small cell lung tumors (SCLC, which constitute about 20-25% of all lung tumors) exhibit a strong expression of 7B2, in contrast to the non-small-cell lung tumors and normal lung cells. Within the small cell lung tumors, variant and classic types can be distinguished. The variant type has lost some neuroendocrine characteristics. We have been able to establish that cells corresponding with the classic type do express 7B2, whereas the variant cell types generally do not exhibit 7B2. This means, therefore, that in particular the 7B2 is a marker for classic small cell lung tumors, and that the presence of classic small cell lung tumors can be established by means of suitable, labelled RNA or DNA probes in hybridisation tests or by means of antisera or monoclonal antibodies, for example, directed against the C-terminal portion of the 7B2 protein, in immunoanalytical methods. This means that, after diagnosis, the small cell lung tumors can be subjected to chemotherapy; non-small-cell lung tumors can generally only be removed by surgery.

In addition, the invention provides the possibility of using 7B2 as a sensitive marker for endocrine tumors (e.g. tumors in the pancreas, the thyroid, the pituitary, the intestine), and to use 7B2 assays in diagnosing brain disorders.

The invention has been realized in a study into the regulation mechanism of exocytosis in neurons and endocrine cells secreting neurotransmitters and hormones. Exocytosis (i.e., the secretory process) means that membranes of so-called secretory granules (vesicles in the cell which contain hormones or neurotransmitters) merged with the cell membrane so that subsequently the hormones and neurotransmitters can be given off to the blood or to another neuron (for communication between neurons in the brain). When this process of exocytosis does not proceed properly, various syndromes may occur as a result of poor information transfer in the brain or as a result of an elevated secretion of certain hormones. Quite recently indications have been obtained to the effect that the nucleotide GTP is involved in the regulation of exocytosis, namely, at the level of membrane fusion. A limited number of proteins have the capacity of binding the nucleotide GTP (GTP-binding proteins). Most of these proteins provide for the transmission of signals obtained through receptors located on the cell membrane, to molecules (enzymes) within the cell (signal transduction). Accordingly, these GTP-binding proteins, which occur in association with the cell membrane and have a molecular weight of 40,000-45,000, are proteins with a very important regulatory function. The proper functioning of a peptide secreting cell requires not only the production of protein precursors for secretory products, but also that the expression of genes associated with the secretory function, such as genes coding for protein precursors processing enzymes and genes coding for proteins involved in the release process. One example of a secretory cell is the melanotroph of the intermediate pituitary gland, which synthesizes proopiomelanocortin (POMC), a prohormone which is processed to form melanophore-stimulating hormone (MSH) among other peptides. The physiological function of the melanotroph cells in amphibians is the synthesis and secretion of MSH in animals on a black background. MSH provides for the process of background adaptation of amphibians by causing dispersion of pigment granules in the skin, as a result of which the animal becomes black on a black background. It is to be expected that proteins involved in the secretion function, like POMC (and hence MSH),are produced in a larger quantity in a black animal than in a white animal. With this in mind, a differential hybridization technique has been carried out to identify a gene associated with POMC gene expression. The differential hybridisation involved the screening of a pituitary cDNA library of the toad Xenopus laevis using single-stranded cDNA probes produced from RNA of induced and non-induced melanotroph cells (i.e., from black and white animals, respectively). One of the differentially hybridizing Xenopus pituitary cDNA clones that did not correspond to POMC mRNA was then used to screen a human pituitary cDNA library under low-stringency hybridization conditions. The nucleotide sequence of two overlapping hybridization-positive human pituitary cDNA clones contains an open reading frame of 633 base pairs, encoding a protein of 211 amino acids (Fig. 1). A computer homology analysis involving a search both at the DNA level (EMBL nucleotide data base; release 11) and the protein level (NBRF/PIR protein data base; release 11) revealed that the amino-terminal region of this protein (amino acid residues 1-77) is identical to the amino-terminal 77 amino acids that have been sequenced for a human pituitary protein of unknown function, called 7B2. No significant homology with any other protein or DNA sequence was found. The cloned cDNA thus codes for the human 7B2 preprotein with an 18-26 amino acid signal peptide sequence for transport across endoplasmic reticulum membranes and a 185 amino acid mature 7B2 protein. It is not yet clear which one of the three methionine residues in the signal peptide functions as the initiator methionine.

The prepeptide of 7B2 is composed of hydrophobic or otherwise uncharged amino acids, except for the Arg residue located in the -23 position, and terminates in the sequence Ala-Phe-Ala; Ala-X-Ala is the most frequently occurring tripeptide preceding a signal peptide cleavage site. The calculated molecular weight of the mature protein ($M_r$ = 20793 Da) agrees with that previously determined for 7B2 by SDS polyacrylamide gel electrophoresis (Mr = 21 kDa).

The data show that the protein contains a cAMP-dependent serine phosphorylation consensus sequence (Arg-Arg-Lys-Arg-Arg-$Ser^{155}$). No potential site for N-linked glycosylation (Asn-X-Ser/Thr) was found. The protein contains three pairs of basic amino acids (the amino acid residues 138-139; 150-154; 171-172) which, in precursor proteins like POMC, are potential cleavage sites of proteolytic enzymes. The 7B2 protein has been highly conserved during 350,000,000 years of vertebrate evolution, as the human and Xenopus proteins exhibit an overall amino acid sequence homology of 83% and, in an amino-terminal region comprising 80 amino acids, as high as 85% homology. A hydropathy analysis of the 7B2 protein shows that, although the amino-terminal portion is more hydrophobic than the carboxy-terminal region, there is no clear membrane-spanning domain in the protein (Fig. 2).

The cloned human 7B2 cDNA was used in Northern blot analysis, whereby a transcript of about 1.35 kb was detected in human pituitary. From this it can be derived that the sequence shown in Fig. 1 represents the virtually complete human 7B2 mRNA sequence (allowing for a poly(A) tail of 100 nucleotides). The presence of two additional minor transcripts of about 1.2 kb and about 0.9 kb suggests the occurrence of 7B2-related mRNA molecules in the human pituitary gland, whereby the transcript of about 1.2 kb could result from the use of the second polyadenylation signal (AATAAA), which is located further to the 5′ end.

Further analysis showed that the 7B2 gene is expressed in a mouse anterior pituitary tumor cell line producing POMC (AtT-20), in rat pheochromocytoma (a chromaffin cell-derived tumor cell line PC-12) and in rat insulinoma (pancreatic tumor cell line RIN-41). Furthermore, 7B2 mRNA can be detected in rat brain stem nucleus, rat brain cortex, and in the supraoptic nucleus and paraventricular nucleus of the rat hypothalamus. On the other hand, no 7B2 mRNA was found in rat pituitary tumor cell line producing growth hormone and prolactin ($GH_4$), and in one mouse (I-10) and two rat (R-2C and LC-540) testicular (Leydig) tumor cell lines. The apparent absence of 7B2 mRNA in the $GH_4$ cells is interesting in that, unlike the pituitary cells from which they are derived, these tumor cells contain only a few, if any, secretory granules and they do not contain substantial amounts of stored hormone. From these observations and from data described earlier in the literature with regard to the tissues in which immuno reactive 7B2 protein has been found, it can be concluded that the 7B2 protein is primarily expressed in cells containing secretory granules, such as neurons and endocrine cells.

An extensive search for amino acid sequence homology with functional domains of regulatory proteins revealed within the 7B2 protein structure the presence of three regions which share significant homology with GTP-binding proteins in domains presumed to participate in GTP binding and hydrolysis (Fig. 3). These regions, indicated as regions A, B and C in Fig. 3,have been assigned on the basis of their conservation within GTP-binding proteins, X-ray crystallographic studies, and site-directed mutagenesis experiments. Region A forms a loop whereby the lysine residue contributes to charge neutralization by ionic contact with a phosphate group of the GDP ligand. The aspartic acid side chain in region B may be involved in magnesium binding. The asparagine residue in region C is situated direct over the guanine ring of the nucleotide. The extensive homology shown in Fig. 3 clearly indicates that the 7B2 protein is structurally related to GTP-binding proteins.

The characteristics of the 7B2 protein (high degree of conservation during vertebrate evolution; its presence in cells with exocytotic secretory processes; localization within secretory granules; presumptive GTP-binding and phosphorylization sites) indicate an important regulatory function, more specifically a possible role in the control of exocytosis. In this connection reference can be made to a recent observation that GTP could regulate exocytosis by acting at two different cellular locations (Barrowman et al., Nature 319 (1986), 504-507). One site is at the level of the plasma membrane, where a family of GTP-binding proteins are known to be involved in the coupling of receptors with their effector systems. The second site of exocytosis control concerns a GTP-regulatory system at the level of secretory granule and plasma membrane fusion. Experiments with botulinum toxin type D, which in adrenal medullary cells ADP-ribosylates an $M_r$ 21,000 protein (and thus deactivates the protein) in parallel with inhibition of exocytosis, indicated that, in this tissue, a putative GTP-binding protein, designated $G_e$ and having a molecular weight of 21000, is associated with exocytosis (Gomperts, Trends biochem. Sci.11, (1986), 290-292, and Burgoyne, Nature 328 (1987), 112-113). The study now made indicates that the $G_e$ protein corresponds to the 21 kDa 7B2 protein.

Accordingly, the study has revealed that the 7B2 is a GTP-binding protein which occurs in association with the membrane of the secretory granule (and not, like other G proteins, with the cell membrane). The 7B2 protein is responsible for the control of exocytosis. In the literature it has been suggested that the ras oncogene product (a 21,000 Dalton GTP-binding protein) could perform this function, but as now shown, this is not the case.

By means of in-situ hybridisation, the 7B2 gene was localized on chromosome 15 (15q13).

The present invention is in the first place embodied in recombinant DNA comprising the gene for protein 7B2 or a significant part thereof, and possibly provided with a detectable label, and in recombinant DNA comprising cDNA for protein 7B2 or a significant part thereof, and possibly provided with a detectable label, and in isolated messenger RNA (mRNA) for protein 7B2, to be obtained from cells in which the protein 7B2 is expressed or from an in vitro transcription system with cDNA for protein 7B2.

Furthermore, the invention is embodied in a method of preparing protein 7B2, which comprises performing an in-vitro or in-vivo translation of recombinant DNA coding for protein 7B2, and isolating the protein 7B2 formed.

The invention further provides monoclonal and polyclonal antibodies having a specificity for protein 7B2, with the exception of antibodies directed against a portion of protein 7B2 consisting of the amino acid residues 23-39.

I prefer antisera induced against a synthetic oligopeptide consisting of the amino acids 173-185. Such an oligopeptide has a high antigenicity, as also have a few other regions in the C-terminal part of protein 7B2.

I have been able to prepare conventional antisera with a specificity for protein 7B2 in, for example, rabbits by standard procedures. Monoclonal antisera could be prepared by the techniques of Köhler and Milstein (Nature 256, 495-497 (1978)). Fusion products of protein 7B2, produced in E.coli K12 could be used as antigen (Grayeb et al.,, EMBO J. 3 2347-2442 (1984) and Molecular Cloning, A Laboratory Manual. T.Maniatis, E.F. Fritsch and J. Sambrook, Cold Spring Harbor Laboratory, 1982).

Finally the invention also comprises a method of detecting 7B2 mRNA or protein in human or animal tissues or body fluids, such as plasma, sputum, brain fluid, urine, and the like by hybridization tests with labelled RNA or DNA probes of protein 7B2, and by immunoanalytic methods, such as immunofluorescence, ELISA, Western blotting, and immunoprecipitation/poly acrylamide gel electrophoresis with antibodies according to the invention.

The invention is described in more detail with reference to the accompanying drawings and a description of the experiments carried out.

In the accompanying drawings, Fig. 1 shows the nucleotide sequence and deduced amino acid sequence of human pituitary cDNA encoding the 7B2 preprotein. Amino acid sequence numbering starts at the amino-terminal amino acid residue of the mature 7B2 protein with the presumptive signal peptide sequence being indicated by negative numbering. The amino acid residues 1-77 are identical to the amino-terminal 77 residues sequenced before for human 7B2. The dot above the serine residue 155 indicates the potential cAMP-dependent protein kinase phosphorylation site. The asterisks denote the translation terminal codon. The two signals for polyadenylation (AATAAA) are overlined.

Fig. 2 shows the hydropathy profile of the human 7B2 preprotein. The profile was plotted using the algorithms of Kyte and Doolittle (J.Molec.Biol. 157 (1982), 105-132) with a window size of 7 amino acid residues. The amino acid residues have been plotted along the abscissa with numbering beginning with the initiator methionine (corresponding to Met at position -26 in Fig. 1). Positive values of the hydropathy denote hydrophobic regions in the protein. The term "signal" indicates the presumed signal peptide sequence. The arrow indicates the position of potential cAMP-dependent protein kinase phosphorylation site (corresponding to the serine at postion 155 in Fig. 1).

Fig. 3 shows a comparison between human 7B2 protein regions and regions within the known GTP-binding proteins. The three regions conserved in GTP-binding proteins, which probably are directly involved in GTB-binding and hydrolysis, are indicated as regions A, B and C. the non-homologous segments separating these three regions are indicated by dotted lines. The amino acid residues Lys, Asp and Asn, shown in capital letters in regions A, B and C, are supposed to be directly involved in GTP-binding and hydrolysis. Groups of identical or conservative amino acid residues are boxed. A gap (-) in region C of the 7B2 protein was introduced to obtain maximum alignment. Numbers of amino acid residues in the 7B2 protein correspond to those of Fig. 1. The sequences of mouse G i, bovine transducin T1, E.coli elongation factor Tu, cKi-ras oncogene product, and yeast GTP-binding protein YP2 have been taken from the literature.

Experimental.

The differential hybridization in the toad Xenopus laevis was carried out in connection with the fact that, on a black background, the melanotroph cells of the pituitary of the toad exhibit a high degree of MSH synthesis and secretion (induced cells) whereas on a white background the expression of the POMC is greatly reduced (non-induced cells). A pituitary cDNA library of Xenopus adapted to black was constructed by introducing size-fractionated and tailled cDNA into the known plasmid pBR 322, also provided with a tail; following the procedure described by Martens et al. in J.Biol.Chem. 260 (1985), 13685-13689. Single-stranded cDNA probes were prepared by oligo(dT) priming of 1,5 µg RNA, isolated from the pars intermedia of toads adapted to black and to white. The differential hybridization comprised a study of 20,000 colonies of the Xenopus pituitary cDNA library with these single-stranded probes under stringent hybridization conditions. One of the differential hybridizing Xenopus pituitary cDNA clones not corresponding met POMC mRNA (clone pX9) was isolated and characterized further. Subsequently, this clone pX9 was used for investigating a human pituitary cDNA library in the gt10 vector. By screening $8x10^5$ clones (standard hybridization solution, 58° C) with pX9, nick-translated to $5x10^8$ cpm per µg DNA, as a probe, 14 hybridization-positive clones were isolated as a result. The nucleotide sequence shown in Fig. 1 was composed from the two overlapping cDNA inserts of the λH6 and λH7 clones; T nucleotide 857 in λH6 is a C nucleotide in λH7. Sequencing of both strands and with overlapping M13 subclones was carried out by the known dideoxy chain termination method described by Sanger et al. in Proc.Natl.Acad.Sci. USA 74 (1977), 5463-5467.

Northern blot analysis of 7B2 mRNA was carried out with RNA samples fractionated on a 1.4% agarose gel after glyoxylation or in 2.2 M formaldehyde. After the transfer, the blots were hybridized in a standard hybridization solution in the presence of 50% formamide with insert DNA of the human pituitary 7B2 cDNA clone H6, obtained by nick-translation ($6x10^8$ cpm per µg DNA). The experiments with glyoxylation were carried out with total human pituitary cellular RNA (25 µg). For the experiments in 2.2 M formaldehyde, the following poly($A^+$) RNA sources were used: rat brain stem nucleus (medulla/pons), 2 µg; mouse AtT-20, 4.3 µg; rat PC-12, 6-h 8-Br-cAMP treatment, 1.4µg; rat PC-12, fresh media, 2µg; rat $GH_4$, 1 µg; rat RIN-41, 5.2 µg; rat R2C, 1.7 µg; rat LC540, 2.8µg; mouse I10, 1.4 µg. The size markers were from an RNA ladder (BRL). The autoradiograms were exposed with two intensifying screens for 20 hours in the case of glyoxylation, and for 9 days in the case of formaldehyde solution at -70 °C. The results are not shown.

There was also carried out a Northern blot analysis of 7B2 mRNA in normal lung tissue and in lung tumors.

Poly($A^+$) samples (10 µg poly ($A^+$)RNA per lane) were glyoxylated and separated on a 1% agarose gel. After transfer to a nylon filter, the filter was hybridized in standard hybridization solution with the 1.2 kB DNA insert of 7B2 cDNA clone H6, marked with $^{32}P$, nick-translated to $5x10^8$ cpm per µg DNA. Lanes 1 and 2: human normal lung, two different individuals; lanes 3 and 4: human small cell lung tumors, two different individuals; lanes 5, 6 and 7: human non-small-cell lung tumors, three different individuals; lanes 8 and 9: feline normal lung.

The marker used was DNA cut with HindIII. The exposure time was 9 days at -70 °C, using two intensification screens. One exposure overnight under these conditions already gave a clear signal in lanes 3 and 4 (not shown). The autoradiogram was overexposed to show the absence of 7B2 mRNA in lanes 1, 2 and 5 to 9 (not shown).

Comparable Northern blot analyses were carried out using cells corresponding to variant and classic small cell lung tumors (results not shown).

Construction of a pGEX-2T-7B2 plasmid

The pGEX-2T-7B2 plasmid expression vector produces in E.coli cells, after induction with the IPTG inducer, a protein which is coupled to the C terminus of the enzyme glutathion S transferase, $M_r$ 26000 (see Smith and Johnson, Gene 67, 1988, 31-40). This vector has the advantage that the fusion protein can easily be purified and that the glutathion S transferase can be removed by proteolysis.

A recombinant plasmid, termed pGEX-2T-7B2, was constructed from the known plasmid pGEX-2T (about 4.9 kb) and a plasmid PAB1, which contains the 7B2 gene. The PAB1 plasmid had been obtained by cloning the 7B2 gene of a human pituitary cDNA clone in a gtl0 library in the EcoRI site of the known pUC18 plasmid. The pB2 gene was situated in an EcoRI-EcoRV fragment, which was isolated by digestion of pAB1 with EcoRI and EcoRV. To ensure a correct reading frame, the plasmid pGEX-2T was treated with BamHI, followed by filling the sticky ends to form blunt ends by means of the Klenow fragment and religation with T4-DNA ligase. The resulting plasmid was treated with SmaI and EcoRI, whereafter the ca. 1 kb EcoRI-EcoRV fragment of pAB1 was added, followed by ligation with T4-DNA ligase. The resulting vector pGEX-2T-7B2 was transformed in RRI E.coli cells.

The expression vector was used for preparing human 7B2 protein in the following manner.

1. Dilute overnight cell culture 1 : 10.
2. Grow this culture at 37 °C for 1 hour.
3. Add 0.5 mM IPTG (induction of tac promotor) and grow at 37 °C for 3 hours.
4.Centrifuge cells at 4000 rpm at 4 °C
5. Resuspend cell pellet in phosphate-buffered saline (pH 7.3) with 1% Trito X-100, 1% Tween-10 and 10 mM Dithiothreitol.
6. Lyse cells by short (30 sec.) sonication of cells.
7. Centrifuge for 5 minutes, 10,000 g, 4 °C.
8. Mix supernatant at room temperature with swollen glutathion agarose (sulphur-coupled, Sigma) for several minutes.
9. Centrifuge 500 g, 10 sec and wash thrice with saline buffer.
10. Elute the fusion protein from the agarose by competition with free glutathion (5 mM glutathion-reduced, Sigma- in 50 mM Tris (pH 8.0) for some minutes.
11. Cleave the purified fusion protein with thrombin (whose cleavage site had been maintained in the plasmid) at 25 °C in elution buffer with 150 mM NaCl, 2.5 mM $CaCl_2$ and 100 ng human thrombin (Sigma) and about 50µg fusion protein (Eaton et al., 1986, Biochemistry 25, 505-512).
12. The cut glutathion and possibly non-cut fusion protein can be removed by absorption to glutathion agarose as described above.
13. The supernatant contains pure human 7B2 (yield up to several mg per liter of culture).

Accordingly, the pGEX-2T-7B2 construct provided large quantities of the human 7B2 protein coupled to glutathion S transferase. Purification of the fusion protein ($M_r$ 47K) was effected by affinity chromatography with glutathion agarose. The 7B2 protein could be liberated from the fusion protein by treatment with the specific protease thrombin. The uncoupled carrier protein (glutathion S transferase) could be removed by absorption of this protein to glutathion agarose. In this way human 7B2 protein could be prepared with facility and on a large scale.

## EXAMPLE

### Detection of 7B2 expression in human long carcinoma cell lines and tumors

According to the criteria of the World Health Organization several classes of human lung cancer can be recognized on histological and morphological grounds: small cell lung carcinoma (SCLC), carcinoid tumor, large cell carcinoma, adenocarcinoma and squamous cell carcinoma. The last three types of carcinomas are also referred to as non-small cell lung carcinoma (NSCLC). In SCLCs, neurosecretory granules are often found. Cell lines derived from SCLC often express numerous properties of the neuroendocrine system including L dopa decarboxylase (DDC), neuron-specific enolase (NSE), the BB isoenzyme of creatine kinase (CK-BB) and peptide hormone bombesin-like immunoreactivity (BLI). This last marker is often referred to as the gastrin-releasing peptide (GRP), the mammalian homologue of bombesin, an amphibian peptide hormone. With few exceptions these markers are not expressed in cell lines of NSCLC. A recent study of a large panel of SCLC cell lines showed that these cell lines can be subdivided into two major categories, i.e., classic (70% of all cell lines) and variant (30%) cell lines (Carney et al., Cancer Res. 45, 1985, 2913-2923. Classic cell lines express elevated levels of DDC, BLI, NSE and CK-BB, whereas variant cell lines only express elevated levels of NSE and CK-BB. The same subdivision can also be made with the use of antisera against intermediate filament proteins. Classic cell lines express cytokeratins, whereas variant cells contain no detectable levels of these intermediate filament proteins. Some of them, however, express neurofilaments and vimentin, which are not detectable in classic cells. Furthermore, variant cell lines often differ morphologically from classic cell lines, they grow faster, contain the c-*myc* oncogene in an amplificated form and exhibit a higher c-*myc* expression level. The variant morphology is sometimes present in some subtypes of primary SCLC tumors and could be associated with the worse prognosis for patients with this type of SCLC.

This example demonstrates the use of 7B2 expression as a marker to discriminate between SCLC cell lines of the classic and the variant type.

Analyses of primary lung cancers indicate that 7B2 expression could be of interest for diagnosis of carcinoid tumors and SLCLS.

### Lung cell lines

The cell lines used were all established human SCLC or NSCLC cell lines. The SCLC cell lines GLC-1-M13 and GLC-1 were obtained from L. de Leij (Cancer Res.45, 1985, 6024-6033 and the SCLC cell lines SCLC-16HC, SCLC-16HV, SCLC-21H and SCLC-22H from G. Bepler (J.Cancer Res.Clin.Oncol. 1113, 1987, 31-40). All other SCLC and NSCLC cell lines (NCI series) were obtained from D.N. Carney (Cancer Res. 45, 1985, 2913-2923).

### Lung carcinomas

Lung carcinoma specimens from 27 patients were selected from the files of the Pathology Department of the St. Antonius Hospital, Niewegein, The Netherlands. Surgical specimens were presented fresh and sterile to the pathologist immediately after extirpation and tumor tissue was quickly frozen in liquid nitrogen and stored at -70° C. Tumors were characterized by routine microscopical and histopathological techniques and classified according to the criteria of the World Health Organization as small cell lung carcinoma (SCLC) (8 cases) or carcinoid tumor (3 cases) or non-small cell lung carcinoma (NSCLC) (16 cases). All SCLCs were examined by electronmicroscopy to confirm the presence of neuroendocrine granules. The NSCLCs could be classified as squamous cell carcinomas (7 cases), adenocarcinomas (8 cases) and in one case the tumor appeared to be of a mixed type. Two specimens of control lung tissue were also obtained.

### RNA isolation and Northern blot analysis

Total cellular RNA was isolated using the lithium-urea procedure described by Auffray and Rougeon (Eur.J.Biochem. 107, 1980, 303-314). Fifteen μg of total RNA was glyoxalated and size fractionated on 1.0% agarose gels and transferred to Hybond-N (procedure as recommended by Amersham). In order to compare similar amounts of total RNA, $OD_{260}$ and ethidium bromide staining were used to estimate RNA concentrations.

DNA probes and hybridization

Probes were isolated and labeled as described by van den Ouwehand et al, Biochim.Biophys.Acta 825, 1985, 140-147. To study 7B2 expression, a 1.2-kbp 7B2-specific cDNA was used as probe. Expression of the gastrin-releasing peptide (GRP) gene was analysed with a 0.9 kbp GRP cDNA probe obtained from Edward A. Sausville (J.Biol.Chem. 261, 1986, 2451-2457). To assay the presence of actin transcripts, pAct-1 a hybrid molecule of pBR322 and 1.2 kbp of actin-specific cDNA sequences of hamster origin (Dodemont et al., EMBO J.1, 1982, 167-171) was used. For hybridizations on nylon membranes (Hybond-N, Amersham) the method of Church and Gilber (PNAS USA 81, 1984, 1991-1995) was used. The Nylon membranes were dehybridized by incubation in 5 mM Tris-HCl (pH 8.0), 2 mM EDTA and 0.1 x Denhardt's solution (1x Denhardt's solution contains 0.02% (w/v) bovine serum albumin, 0.02% (w/v) polyvinylpyrrolidone and 0.02% (w/v) ficoll)) at 65°C for 2 hr. Each dehybridization was checked by autoradiography. Blots could be used several times without significant loss of signal.

Espression of 7B2 in human lung carcinoma cell lines

A 1.2 kbp cDNA probe encompassing all the coding sequences for the 7B2 protein was used as a molecular probe in Northern blot analyses. With this probe, a 1.35 kb 7B2-specific mRNA was found in all nine tested SCLC cell lines of the classic type (SCLC-c), whereas in the SCLC cell lines of the variant type (SCLC-v) only in one out of seven expression of the 7B2 gene could be detected (see Table 1). In NSCLC cell lines, which in general have no neuroendocrine characteristics, only in one cell line (NCI-H441) 7B2 expression was found. Expression of 7B2 was also compared with expression of the gastrin-releasing peptide (GRP) gene which encodes an 0.8 kb mRNA. This GRP gene is often used as a marker to discriminate between SCLC-c cell lines, which in general show expression of GRP, and SCLC-v and NSCLC cell lines, which have generally only low or no expression of GRP. After dehybridization, the same Northern blots as used for analysing 7B2 expression were analysed with a GRP cDNA probe. No expression of GRP was found in cell lines which had no 7B2 expression, only in three cell lines with 7B2 expression (two SCLC-c and one NSCLC) GRP was not co-expressed (see Table 1). Finally, after a second dehybridization the blots were again used to check the amount and quality of the total RNA samples on the Northern blots. Analyses with a actin-specific probe showed that differences in amount or quality of the total mRNA samples could not account for differences in 7B2 or GRP expression found in the analyzed lung carcinoma cell lines (results not shown).

Expression of 7B2 in human lung tumors

The results of the expression studies of 7B2 in human lung carcinoma cell lines indicated that similar analyses of primary human lung tumors could be of interest. A total of 27 lung tumors, all classified according to the criteria of the World Health Organization were analyzed for 7B2 expression. These analyses included two control lung tissue specimens. The results of a Northern blot analysis of similar amounts of total RNA from the various types of lung tumors and control lung tissue are not shown. The conditions were the same in all experiments and exposure of the film was 36 h (using Kodak XAR-5 and two intensifying screens (Lightning Plus; Dupont Photo Products)). The level of 7B2 expression was classified into four categories, including very high, high, low, and very low/not detectable under the above mentioned conditions (see Table 2). A discrimination between very low and not detectable 7B2 expression levels was more easily made after exposure times of more than a week: in the control lung tissues no 7B2 expression was detected, whereas in NSCLCs a very low 7B2 expression could be detected in a part of the tumor specimens (data not shown). The autoradiographs were scanned with a laser scanner to estimate the differences in expression levels. The ratio of expression of 7B2 for the very high, high and low expression categories was calculated to be about 16 : 4 : 1. Very low expression was estimated to be 10 to 25% of the low expression level. The minor differences in the amount of the total RNA samples as analyzed by hybridization with a actin-specific probe were taken into account. In Table 2 the results of the 7B2 expression analyses are shown. In control lung tissue the 1.35 kb 7B2 mRNA is not detectable. In SCLC the expression of 7B2 was variable. Three specimens of SCLC showed high levels of expression, whereas three other specimens had low expression levels. In the two remaining cases expression was very low or not detectable: longer exposure showed in one case a very low expression whereas in the other no expression could be detected. All three specimen of carcinoid tumors appeared to have very high levels of 7B2 expression. In the NSCLCs only very low or not detectable levels of 7B2 expression were found. Longer exposure revealed a possible tendency that the very low levels of expression of 7B2 detectable in NSCLCs is present in adenocarcinomas rather than in squamous cell carcinomas.

TABLE 1

Expression of the gene encoding the 7B2 protein in human lung cancer cell lines.

| Cell line | Classification | 7B2 expression | GRP expression |
|---|---|---|---|
| GLC-1-M13 | SCLC-c | + | + |
| SCLC-H16C | SCLC-c | + | - |
| SCLC-H21 | SCLC-c | + | - |
| SCLC-H22 | SCLC-c | + | + |
| NCI-H209 | SCLC-c | + | + |
| NCI-H249 | SCLC-c | + | + |
| NCI-H345 | SClC-c | + | + |
| NCI-H510 | SCLC-c | + | + |
| NCI-H592 | SCLC-c | + | + |
| GLC-1 | SCLC-v | - | - |
| SCLC-H16V | SCLC-v | - | - |
| NCI-H82 | SCLC-v | - | - |
| NCI-N417 | SCLC-v | - | - |
| NCI-H446 | SCLC-v | - | - |
| NCI-H524 | SCLC-v | - | - |
| NCI-H526 | SCLC-v | + | - |
| NCI-H125 | NSCLC | - | - |
| NCI-H441 | NSCLC | + | - |
| NCI-H460 | NSCLC | - | - |
| NCI-H596 | NSCLC | - | - |

TABLE 2

Expression of the gene encoding the 7B2 protein in specimens of normal human lung tissue and in primary human lung tumors

| Human lung tissue | Total numbers of specimens tested | Numbers in different categories of 7B2 expression*: very high | high | low | very low/not detectable |
|---|---|---|---|---|---|
| SCLC | 8 | - | 3 | 3 | 2 |
| Carcinoid tumor | 3 | 3 | - | - | - |
| Squamous cell carcinoma | 7 | - | - | - | 7 |
| Adenocarcinoma | 8 | - | - | - | 8 |
| Adeno/squamous cell carcinoma | 1 | - | - | - | 1 |
| Control lung | 2 | - | - | - | 2 |

* the quantitative classification of the different expression categories is discussed in the text.

SUMMARY OF THE RESULTS

Using 7B2-specific cDNA as a probe, both tumor specimens of primary human lung carcinomas and cell lines derived from these have been investigated for expression of 7B2. Northern blot analyses showed that in all 9 small cell lung carcinoma cell lines of the classic type (SCLC-C) tested 7B2 is expressed. In all small cell lung carcinoma cell lines of the variant type (SCLC-V) tested, however, expression of 7B2 was not detected or hardly so. In the analysis of non-small cell lung carcinoma (NSCLC) cell lines, 7B2 expression was hardly, if at all, detectable. In primary SCLC tumors, 4 tumors were found to be highly positive, 2 moderately positive and two hardly positive. In primary NSCLC tumors, the expression of 7B2 was also hardly detectable. Finally, carcinoids were found to exhibit very high 7B2 expression.

## Claims

1. Recombinant DNA comprising the gene for protein 7B2 or a significant part thereof, and possibly provided with a detectable label.

2. Recombinant DNA comprising cDNA for protein 7B2 or a significant part thereof, and possibly provided with a detectable label.

3. Isolated messenger RNA (mRNA) for protein 7B2, obtainable from cells in which the protein 7B2 is expressed or from an in vitro transcription system containing cDNA for protein 7B2.

4. A process for preparing protein 7B2, which comprises an in-vitro or in-vivo translation of recombinant DNA coding for protein 7B2, and isolating the protein 7B2 formed.

5. Monoclonal and polyclonal antibodies with a specificity for protein 7B2, except for antibodies directed against a portion of protein 7B2 consisting of the amino acid residues 23-39.

6. A method of detecting 7B2 mRNA or protein in human or animal tissues or body fluid, such as plasma, sputum, brain fluid, urine, and the like, by hybridization tests with labelled RNA or DNA probes of protein 7B2, or by immunoanalytic methods such as immunofluorescence, ELISA, Western blotting, and immunoprecipitation/polyacrylamide gel elctrophoresis with antibodies as claimed in claim 5.

# FIG.1

```
                                                 5'----CGCTCCTCGGGCTGCCCCTCGGTTGACA  28

-26                 -20                                     -10                     -1
Met Val Ser Arg Met Val Ser Thr Met Leu Ser Gly Leu Leu Phe Trp Leu Ala Ser Gly Trp Thr Pro Ala Phe Ala
ATG GTC TCC AGG ATG GTC TCT ACC ATG CTA TCT GGC CTA CTG TTT TGG CTG GCA TCT GGA TGG ACT CCA GCA TTT GCT  106

  1                         10                                      20
Tyr Ser Pro Arg Thr Pro Asp Arg Val Ser Glu Ala Asp Ile Gln Arg Leu Leu His Gly Val Met Glu Gln Leu Gly
TAC AGC CCC CGG ACC CCT GAC CGG GTC TCA GAA GCA GAT ATC CAG AGG CTG CTT CAT GGT GTT ATG GAG CAA TTG GGC  184

            30                          40                                      50
Ile Ala Arg Pro Arg Val Glu Tyr Pro Ala His Gln Ala Met Asn Leu Val Gly Pro Gln Ser Ile Glu Gly Gly Ala
ATT GCC AGG CCC CGA GTG GAA TAT CCA GCT CAC CAG GCC ATG AAT CTT GTG GGC CCC CAG AGC ATT GAA GGT GGA GCT  262

                    60                                      70
His Glu Gly Leu Gln His Leu Gly Pro Phe Gly Asn Ile Pro Asn Ile Val Ala Glu Leu Thr Gly Asp Asn Ile Pro
CAT GAA GGA CTT CAG CAT TTG GGT CCT TTT GGC AAC ATC CCC AAC ATC GTG GCA GAG TTG ACT GGA GAC AAC ATT CCT  340

    80                          90                                      100
Lys Asp Phe Ser Glu Asp Gln Gly Tyr Pro Asp Pro Pro Asn Pro Cys Pro Val Gly Lys Thr Asp Asp Gly Cys Leu
AAG GAC TTT AGT GAG GAT CAG GGG TAC CCA GAC CCT CCA AAT CCC TGT CCT GTT GGA AAA ACA GAT GAT GGA TGT CTA  418

                    110                                 120                                     130
Glu Asn Thr Pro Asp Thr Ala Glu Phe Ser Arg Glu Phe Gln Leu His Gln His Leu Phe Asp Pro Glu His Asp Tyr
GAA AAC ACC CCT GAC ACT GCA GAG TTC AGT CGA GAG TTC CAG TTG CAC CAG CAT CTC TTT GAT CCG GAA CAT GAC TAT  496

                                140                                 150                     •
Pro Gly Leu Gly Lys Trp Asn Lys Lys Leu Leu Tyr Glu Lys Met Lys Gly Gly Glu Arg Arg Lys Arg Arg Ser Val
CCA GGC TTG GGC AAG TGG AAC AAG AAA CTC CTT TAC GAG AAG ATG AAG GGA GGA GAG AGA CGA AAG CGG AGG AGT GTC  574

            160                                     170                                 180
Asn Pro Tyr Leu Gln Gly Gln Arg Leu Asp Asn Val Val Ala Lys Lys Ser Val Pro His Phe Ser Asp Glu Asp Lys
AAT CCA TAT CTA CAA GGA CAG AGA CTG GAT AAT GTT GTT GCA AAG AAG TCT GTC CCC CAT TTT TCA GAT GAG GAT AAG  652

Asp Pro Glu ***
GAT CCA GAG TAA AGAGAAGATGCTAGACGAAAACCCACATTACCTGTTAGGCCTCAGCATGGCTTATGTGCACGTGTAAATGGAGTCCCTGTGAATGAC  751

AGCATGTTTCTTACATAGATAATTATGGATACAAAGCAGCTGTATGTAGATAGTGTATTGTCTTCACACCGATGATTCTGCTTTTTGCTAAATTAGAATAAGA  854

GCTTTTTTGTTTCTTGGGTTTTTAAAATGTGAATCTGCAATGATCATAAAAATTAAAATGTGAATGTCAACAATAAAAAGCAAGACTATGAAAGGCTCAGATT  957

TCTTGCAGTTTAAAATGGTGTCTGAGGTTGTACTATTTTGGCCAAGTCTGTAGAAAGCTGTCATTTGATTTTGATTATGTAGTTCATCCAGCCCTTGGGCATT  1060

GTTATACACCAGTAAAGAAGGCTGTACTCAAGAGGAGGAGCTGACACATTTCACTTGGCTGCGTCTTAATAAACATGAATGCAAGCATTGGC(A)n----3'  1152
```

FIG.2

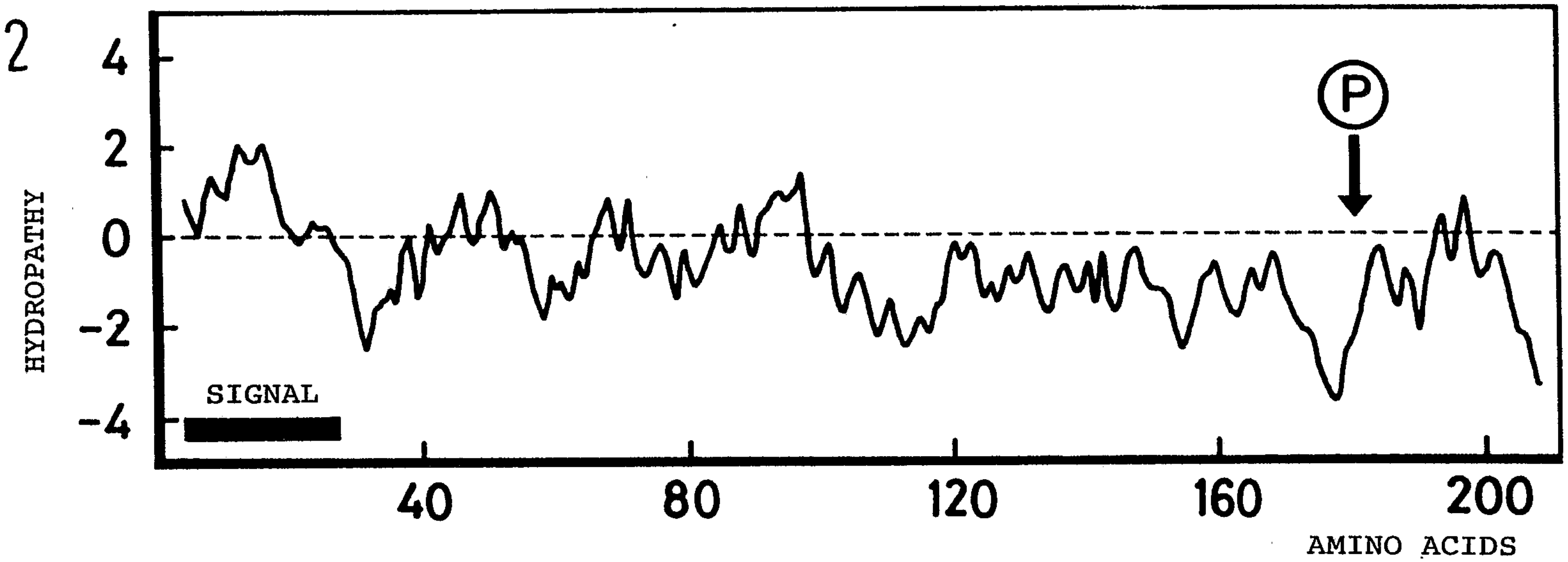
HYDROPATHY
4
2
0
-2
-4
SIGNAL
P
40
80
120
160
200
AMINO ACIDS

FIG.3

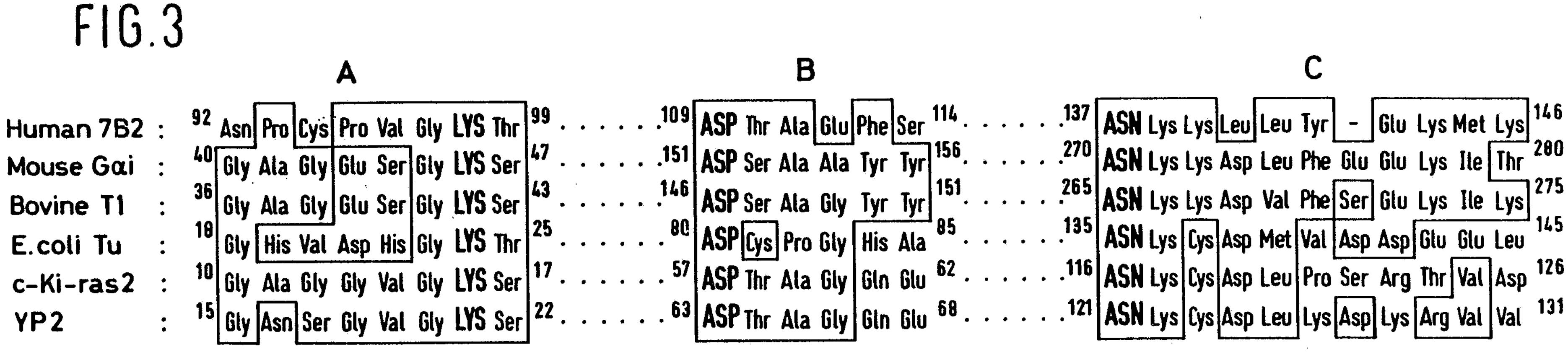
A
B
C
Human 7B2 : 92 Asn Pro Cys Pro Val Gly LYS Thr 99 ...... 109 ASP Thr Ala Glu Phe Ser 114 ...... 137 ASN Lys Lys Leu Leu Tyr - Glu Lys Met Lys 146
Mouse Gαi : 40 Gly Ala Gly Glu Ser Gly LYS Ser 47 ...... 151 ASP Ser Ala Ala Tyr Tyr 156 ...... 270 ASN Lys Lys Asp Leu Phe Glu Glu Lys Ile Thr 280
Bovine T1 : 36 Gly Ala Gly Glu Ser Gly LYS Ser 43 ...... 146 ASP Ser Ala Gly Tyr Tyr 151 ...... 265 ASN Lys Lys Asp Val Phe Ser Glu Lys Ile Lys 275
E.coli Tu : 18 Gly His Val Asp His Gly LYS Thr 25 ...... 80 ASP Cys Pro Gly His Ala 85 ...... 135 ASN Lys Cys Asp Met Val Asp Asp Glu Glu Leu 145
c-Ki-ras2 : 10 Gly Ala Gly Gly Val Gly LYS Ser 17 ...... 57 ASP Thr Ala Gly Gln Glu 62 ...... 116 ASN Lys Cys Asp Leu Pro Ser Arg Thr Val Asp 126
YP2 : 15 Gly Asn Ser Gly Val Gly LYS Ser 22 ...... 63 ASP Thr Ala Gly Gln Glu 68 ...... 121 ASN Lys Cys Asp Leu Lys Asp Lys Arg Val Val 131

European Patent Office

# EUROPEAN SEARCH REPORT

Application Number

EP 88 20 2395

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | THE JOURNAL OF BIOCHEMISTRY AND CYTOCHEMISTRY, vol. 33, no. 12, December 1985, pages 1219-1226, The Histochemical Society, Inc., New York, US; M. MARCINKIEWICZ et al.: "Immunocytochemical localization of a novel pituitary protein (7B2) within the rat brain and hypophysis"<br>* Whole article *<br>--- | 1-5 | C 12 N 15/00<br>C 12 P 21/02<br>C 12 P 21/00<br>G 01 N 33/577 |
| Y,D | ARCHIVES OF BIOCHEMISTRY AND BIOPHYSICS, vol. 225, no. 2, September 1983, pages 525-534, Academic Press, Inc., New York, US; N.G. SEIDAH et al.: "Isolation and NH2- terminal sequence of a highly conserved human and porcine pituitary protein belonging to a new superfamily"<br>* Whole article *<br>--- | 1-5 | |
| Y | GENETIC ENGINEERING, vol. 2, 1980, pages 31-46, London, GB; S.-P. KWAN et al.: "Production of monoclonal antibodies"<br>* Whole article *<br>--- | 5 | **TECHNICAL FIELDS SEARCHED (Int. Cl.4)**<br>C 12 N<br>C 12 P |
| Y,D | JOURNAL OF BIOLOGICAL CHEMISTRY, vol. 260, no. 25, 5th November 1985, pages 13685-13689, The American Society of Biological Chemists, Inc., Baltimore, US; G.J.M. MARTENS et al.: "Nucleotide sequence of cloned cDNA for pro-opiomelanocortin in the amphibian xenopus laevis"<br>* Whole article *<br>--- -/- | 1-4 | |

The present search report has been drawn up for all claims

| Place of search | Date of completion of the search | Examiner |
|---|---|---|
| THE HAGUE | 05-01-1989 | VAN PUTTEN A.J. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

European Patent Office

# EUROPEAN SEARCH REPORT

Application Number

EP 88 20 2395

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl.4) |
| P,X | FEBS LETTERS, vol. 234, no. 1, 4th July 1988, pages 160-164, Federation of European Biochemical Societies, Amsterdam, NL; G.J.M. MARTENS: "Cloning and sequence analysis of human pituitary cDNA encoding the novel polypeptide 7B2<br>* Whole article *<br>----- | 1-5 | |
| | | | TECHNICAL FIELDS SEARCHED (Int. Cl.4) |

The present search report has been drawn up for all claims

| Place of search | Date of completion of the search | Examiner |
|---|---|---|
| THE HAGUE | 05-01-1989 | VAN PUTTEN A.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)